# EUROPÄISCHE PATENTSCHRIFT

(11) **EP 2 427 768 B1**
(45) Veröffentlichungstag und Bekanntmachung des Hinweises auf die Patenterteilung: **06.03.2013**
(21) Anmeldenummer: 10717057.3
(22) Anmeldetag: 24.04.2010
(51) Int. Cl.: G01N 33/543

(54) **VERFAHREN ZUM NACHWEIS DES VORHANDENSEINS VON MOLEKÜLEN MITTELS OPTISCHER GITTER**
METHOD FOR DEMONSTRATING THE PRESENCE OF MOLECULES BY MEANS OF OPTICAL GRATINGS
PROCÉDÉ POUR DÉTECTER LA PRÉSENCE DE MOLÉCULES AU MOYEN DE RÉSEAUX OPTIQUES

(30) Priorität: 05.05.2009 DE 102009019717
(43) Veröffentlichungstag der Anmeldung: 14.03.2012
(73) Patentinhaber: Karlsruher Institut für Technologie, 76131 Karlsruhe (DE)
(72) Erfinder: WALHEIM, Stefan, 76356 Weingarten (DE); SCHIMMEL, Thomas, 76131 Karlsruhe (DE); LENHERT, Steven, Tallahasse Florida 32301 (US); FUCHS, Harald, 48145 Münster (DE); BRINKMANN, Falko, 22305 Hamburg (DE); LAUE, Thomas, 76344 Eggenstein-Leopoldshafen (DE)
(86) Internationale Anmeldenummer: PCT/EP2010/002534
(87) Internationale Veröffentlichungsnummer: WO 2010/127780

(56) Entgegenhaltungen:
- WO-A2-2008/156732
- LENHERT STEVEN ET AL: "Lipid multilayer gratings." April 2010 (2010-04), NATURE NANOTECHNOLOGY APR 2010 LNKD- PUBMED:20190751, VOL. 5, NR. 4, PAGE(S) 275 - 279 , XP002587198 ISSN: 1748-3395 das ganze Dokument
- SEKULA SYLWIA ET AL: "Multiplexed lipid dip-pen nanolithography on subcellular scales for the templating of functional proteins and cell culture" SMALL, JOHN WILEY AND SONS, WEINHEIM AN DER BERGSTRASSE, GERMANY LNKD- DOI:10.1002/SMLL.200800949, Bd. 4, Nr. 10, 1. Oktober 2008 (2008-10-01), Seiten 1785-1793, XP002564135 ISSN: 1613-6829 [gefunden am 2008-09-22]
- LENHERT STEVEN ET AL: "Massively parallel dip-pen nanolithography of heterogeneous supported phospholipid multilayer patterns." SMALL (WEINHEIM AN DER BERGSTRASSE, GERMANY) JAN 2007 LNKD- PUBMED:17294472, Bd. 3, Nr. 1, Januar 2007 (2007-01), Seiten 71-75, XP002587199 ISSN: 1613-6829
- SALAITA KHALID ET AL: "Applications of dip-pen nanolithography" NATURE NANOTECHNOLOGY, NATURE PUBLISHING GROUP, GB LNKD- DOI:10.1038/NNANO.2007.39, Bd. 2, Nr. 3, 1. März 2007 (2007-03-01), Seiten 145-155, XP002564136 ISSN: 1748-3395 [gefunden am 2007-05-25]
- KUMAR A ET AL: "Patterned Condensation Figures as Optical Diffraction Gratings" SCIENCE, AMERICAN ASSOCIATION FOR THE ADVANCEMENT OF SCIENCE, WASHINGTON, DC; US LNKD- DOI:10.1126/SCIENCE.263.5143.60, Bd. 263, 7. Januar 1994 (1994-01-07), Seiten 60-62, XP002555160 ISSN: 0036-8075

## Beschreibung

Die Erfindung betrifft ein Verfahren zum Nachweis des Vorhandenseins von Molekülen, insbesondere von Biomolekülen, die sich in einer flüssigen Phase befinden.

Der Nachweis von Molekülen, insbesondere von Biomolekülen in Flüssigkeiten, gewinnt zunehmend an wirtschaftlicher Bedeutung, insbesondere in Biologie, Chemie, Biochemie, Pharmazie sowie medizinischer Diagnostik und Forschung.

Für den praktischen Einsatz eines erfindungsgemäßen Verfahrens sind die folgenden Merkmale entscheidend:
- schneller und spezifischer Nachweis;
- markerfreier (*label-free*) Nachweis;
- Möglichkeit der Verfolgung zeitabhängiger Vorgänge in Echtzeit, z.B. zur Untersuchung der Kinetik von (bio-)chemischen Prozessen;
- Eignung unter reaktiven Bedingungen;
- Biokompatibilität; und
- Verzicht auf toxische Komponenten.

Für den praktischen Einsatz einer erfindungsgemäßen Vorrichtung sind die folgenden Merkmale entscheidend:
- leichte Handhabung;
- kostengünstige Implementierung;
- kompaktes Design, vorzugsweise Miniaturisierbarkeit bis in den Mikrometerbereich, d.h. auf Längenskalen im Bereich oder kleiner als die Abmessungen von biologischen Zellen;
- Möglichkeit des Nachweises innerhalb von geschlossenen (bio-) chemischen (Reaktions-)Gefäßen;
- Integrierbarkeit zu Arrays vieler, vorzugsweise für unterschiedliche Substanzen spezifisch reagierender Sensoren, die sich durch dieselbe Auslesemimik auslesen lassen;
- Möglichkeit des schnellen sequentiellen bzw. parallelen Auslesens vieler Sensoren, insbesondere über Vielkanaldetektoren oder mittels Multiplexing;
- Möglichkeit der in-situ-Untersuchung.

Ivnitski, D., Abdel-Hamid, I., Atanasov, P. und Wilkins, E., *Biosensors for detection of pathogenic bacteria*, Biosens. Bioelectron. 14, S. 599, 1999, klassifizieren markerfreie (*label-free*) Biosensoren in physikalischer Hinsicht in drei Gruppen:
1. Massensensitive Sensoren mit piezoelektrischen Materialien oder Cantilevern zum Nachweis des Vorhandenseins von biologischem Material;
2. Elektrochemische Sensoren, die auf der Messung des Stroms oder Widerstands eines Transducers basieren; und
3. Optische Sensoren, die die Änderung des Brechungsindex oder das Vorhandensein einer dünnen Schicht nachweisen.

Aus der US 7,118,710 B2 ist ein Biosensor bekannt, der ein auf ein Substrat aufgebrachtes zweidimensionales Gitter mit einem hohen Brechungsindex und ein oder mehrere spezifische bindende markerfreie Substanzen umfasst, die auf der Oberfläche des Gitters immobilisiert sind. Bei Beleuchtung des Biosensors zeigt sich eine Veränderung der Beugungsintensität.

Die WO 2000/041213 A1 beschreibt *Dip-Pen Nanolithography* (DPN) als lithographisches Verfahren zur Herstellung von Strukturen mit Abmessungen von 10-1000 nm durch ein Rasterkraftmikroskop, dessen Spitze mit einer Lösung (Tinte) benetzt, die durch eine treibende Kraft auf die Oberfläche eines Substrats übertragen wird. Aufgrund der Luftfeuchtigkeit bildet sich zwischen Spitze und Oberfläche eines Substrats ein wässriger Meniskus aus, der dem Transfer der Moleküle von der Spitze auf die Oberfläche dient, wo die Moleküle chemisch absorbiert werden oder sich auf entsprechend präparierten Oberflächen anordnen.

Die EP 1881368 A2 offenbart den Einsatz von Membranlipiden als Tinte für DPN, wobei die Anzahl der Lipid-(Doppel-)schichten auf der Oberfläche des Substrats über die Geschwindigkeit der Spitze des Rasterkraftmikroskops über der Oberfläche des Substrats und die relative Luftfeuchtigkeit in der Atmosphäre einstellbar ist.

In S. Sekula, J. Fuchs, S. Weg-Remers, P. Nagel, S. Schuppler, J. Fragala, N. Theilacker, M. Franzreb, C. Wingren, P. Ellmark, C.A.K. Borrebaeck, C.A. Mirkin, H. Fuchs und S. Lenhert, Multiplexed Lipid Dip-Pen Nanolithography on Subcellular Scales for the Templating of Functional Proteins and Cell Culture, Small 4, S. 1785-1793, 2008, werden der Einsatz von DPN zur Herstellung von Mikro- und Nanostrukturen aus flüssigen Membranlipiden, die verschiedene Anteile an Makromolekülen enthalten, und deren Charakterisierung durch Fluoreszenz- und Photoemissionselektronenmikroskopie beschrieben.

A. Kumar und G. Whitesides, Science 263, S. 60, 1994, beschreiben die Kondensation von Wasser auf einer selbstorganisierten Monolage von ω-funktionalisierten Alkanthiolaten auf Gold. Diese Kondensationsfigur bildet ein optisches Brechungsgitter für reflektiertes oder transmittiertes Licht einer Wellenlänge von 632,8 nm, das einer Atmosphäre mit einer konstanten relativen Luftfeuchtigkeit ausgesetzt wird.

Ausgehend hiervon ist es die Aufgabe der vorliegenden Erfindung, ein Verfahren zum Nachweis des Vorhandenseins von Molekülen, insbesondere von Biomolekülen, die sich in einer flüssigen Phase befinden, vorzuschlagen, die die vorher genannten Nachteile und Einschränkungen nicht aufweisen.

Insbesondere soll ein Verfahren bereitgestellt werden, das den schnellen, spezifischen markerfreien Nachweis sowohl von niedermolekularen Molekülen als auch von Biomolekülen ermöglicht.

Diese Aufgabe wird durch die Schritte des Anspruchs 1 gelöst. Die Unteransprüche beschreiben vorteilhafte Ausgestaltungen der Erfindung.

Das erfindungsgemäße Verfahren zum Nachweis des Vorhandenseins eines Ananlyts in Form von Molekülen umfasst die Schritte a) bis c).

Zunächst wird gemäß Schritt a) mindestens ein optisches Beugungsgitter, das aus einer ersten flüssigen Phase besteht, bereitgestellt. Die flüssige Phase liegt auf einem Substrat in Form von periodischen Strukturen, insbesondere von Linien, Punkten oder Quadraten, die eine Periodizität von 190 nm bis 10 µm aufweisen, vor. Unterhalb von 190 nm verringert sich die Transmission der Luft derart, dass der optische Nachweis gemäß Schritt c) praktisch nicht mehr möglich ist, während sich an Strukturen mit einer Periodizität oberhalb von 10 µm keine Farbeffekte mehr beobachten lassen.

Das feste Substrat besteht bevorzugt aus Glas, Silizium, einem Polymer, bevorzugt Polymethylmethacrylat(PMMA), oder einem Metall, insbesondere aus Gold oder Silber.

In einer besonderen Ausgestaltung wird die Oberfläche des Substrats zuvor funktionalisiert, insbesondere durch selbstorganisierte Monolagen, Plasmabehandlung oder Abscheidung aus der Dampfphase. Insbesondere erfolgt die Funktionalisierung des Substrats derart, dass sich auf diese Weise auf der Oberfläche verschiedene Bereiche ergeben, so dass auf demselben Substrat aufgebrachte optische Gitter sich auf Oberflächen mit unterschiedlichen Eigenschaften befinden.

In einer bevorzugten Ausgestaltung wird das optische Gitter mittels der *Dip-Pen Nanolithographie* (DPN) hergestellt. Mit diesem Verfahren werden bevorzugt Membranlipide, vorzugsweise Phospholipide, auf das Substrat aufgebracht.

Bevorzugte Phospholipide sind 1,2-dioleoyl-sn-glycero-3-phosphocholin, kurz DOPC, 1,2-dipalmitoyl-sn-glycero-3-phosphoethanolamine-N-(cap Biotinyl), kurz Biotin-Lipid, oder das Nickelsalz des 1,2-Dioleoyl-sn-Glycero-3-{[N(5-Amino-1-arboxypentyl)iminodiacetosäuresuccinyls, kurz NTA Lipid.

In einer alternativen Ausgestaltung wird das optische Gitter durch strukturierte Entnetzung der ersten flüssigen Phase auf dem Substrat, das in seinen Benetzungseigenschaften lateral strukturiert ist, hergestellt. Hierfür eignet sich auf dem Substrat das Vorliegen einer Oberflächenrauheit (Kerben oder Rauhigkeitskontrast), von chemischen Heterogenitäten oder von geometrischen Strukturen, woran sich die erste flüssige Phase durch so genanntes *Pinning* festhält.

Zur Herstellung eines derartigen optischen Gitters wird eine Flüssigkeit oder eine verdünnte Lösung einer Flüssigkeit auf das in seinen Benetzungseigenschaften lateral strukturierte Substrat aufgebracht. Dies erfolgt durch parallele und serielle Verfahren, bevorzugt Stempelverfahren, insbesondere Mikrokontaktdrucken, Prägeverfahren, Elektronenstrahl-, Photo- oder Laserinterferenzlithographie, direktes Schreiben mit der Spitze eines Kraftmikroskops oder einem Laserstrahl. Die benetzbaren Bereiche werden anschließend von der Flüssigkeit bedeckt, wodurch sich auf dem Substrat das periodische Muster ausbildet, das aus der ersten flüssigen Phase besteht.

In besonderen Ausgestaltungen werden zur ersten flüssigen Phase, woraus das mindestens eine optische Gitter besteht, Additive hinzugefügt, vorzugsweise Cholesterin zur Einstellung der Viskosität und der Permeabilität der Gitterstrukturen. Weitere Additive sind transmembrane oder periphere Membranlipide, einschließlich natürlicher oder synthetisch hergestellter Proteine für spezifische Funktionalitäten.

Das so bereitgestellte mindestens eine optische Gitter weist optische Eigenschaften auf, die durch die Gegenwart der nachzuweisenden molekularen Spezies verändert werden können. Die hierfür bevorzugten Eigenschaften eines optischen Gitters sind
- der Brechungsindex und der Verlauf des Brechungsindex als Funktion des Ortes längs des Querschnittes des Gitters,
- die Reflektivität des Gitters,
- die Absorptionseigenschaften des Gitters,
- der Verlauf der Intensität I(α) als Funktion des Winkels α,
- der Verlauf der Intensität I(λ) als Funktion der Wellenlänge λ bei Reflexion oder Transmission von Licht am optischen Gitter.

Das bereitgestellte optische Beugungsgitter wird weiter gemäß Schritt a) mit einer zweiten flüssigen Phase, die nicht mit der ersten flüssigen Phase mischbar ist, in Kontakt gebracht. Auf diese Weise bildet sich zwischen dem optischen Gitter, das aus einer ersten periodisch angeordneten flüssigen Phase besteht, und der zweiten flüssigen Phase eine Flüssig-Flüssig-Grenzschicht (*liquid-liquid interface*) aus.

In einer bevorzugten Ausgestaltung bestehen die Strukturen des optischen Gitters aus einem Membranlipid und als zweite flüssige Phase wird eine wässrige Lösung, worin sich die nachzuweisenden Moleküle befinden, eingesetzt.

In einer besonderen Ausgestaltung wird das mindestens eine optische Gitter vor dem Inkontaktbringen mit der zweiten flüssigen Phase in ein getrocknetes Gas, vorzugsweise einem Inertgas, eingebracht.

Gemäß Schritt b) werden die nachzuweisenden Moleküle, die auch ionisiert sein können, vor, während oder nach Schritt a) der ersten flüssigen Phase, woraus das mindestens eine optische Gitter besteht, oder der zweiten flüssigen Phase zugegeben.

In einer bevorzugten Ausgestaltung enthält die zweite flüssige Phase die nachzuweisenden Moleküle, die die Fähigkeit besitzen, die optischen Eigenschaften des optischen Gitters zu verändern, bevor die zweite flüssige Phase mit dem optischen Gitter in Kontakt gebracht wird.

In einer alternativen bevorzugten Ausgestaltung wird die zweite flüssige Phase zunächst gemäß Schritt a) mit dem optischen Gitter in Kontakt gebracht, bevor anschließend in die zweite flüssige Phase die nachzuweisenden Moleküle, die die Fähigkeit besitzen, die optischen Eigenschaften des optischen Gitters zu verändern, zugegeben werden.

In einer weiteren Ausgestaltung werden die nachzuweisenden Moleküle zunächst zusammen mit der ersten flüssigen Phase als Bestandteil in das optische Gitter gemäß Schritt a) eingebaut. Das optische Gitter, worin bereits die nachzuweisenden Moleküle enthalten sind, wird erst anschließend mit der zweiten flüssigen Phase in Kontakt gebracht.

Unabhängig von der Art und Weise der Zuführung der nachzuweisenden Moleküle erfolgt der Nachweis der nachzuweisenden Moleküle erst dann, wenn die nachzuweisenden Moleküle sowohl mit der zweiten flüssigen Phase als auch unmittelbar mit der ersten flüssigen Phase des optischen Gitters zusammentreffen.

Die spezielle Fähigkeit der nachzuweisenden Moleküle, die optischen Eigenschaften des optischen Gitters zu verändern, ist Voraussetzung dafür, dass der während Schritt c) erfolgende Nachweis der molekularen Spezies über eine Veränderung der Intensität oder des räumlichen Verlauf des Beugungsmusters ermöglicht wird.

Zur Durchführung des erfindungsgemäßen Verfahrens eignen sich insbesondere Moleküle, die die Fähigkeit aufweisen, die geometrische Form, die optische Dichte bzw. die Absorptionseigenschaften des optischen Gitters zu verändern.

In einer bevorzugten Ausgestaltung werden Moleküle eingesetzt, die die Grenzflächenenergie des optischen Gitters unter einer umgebenden Flüssigkeit ändern und damit vorzugsweise auf dem Substrat Entnetzungsvorgänge hervorrufen.

In einer weiteren Ausgestaltung werden Moleküle eingesetzt, die durch Änderung des Benetzungswinkels der Linien des Gitters zum Substrat die Form des Gitters und somit dessen optische Eigenschaften ändern.

In einer weiteren Ausgestaltung werden Moleküle eingesetzt, die mit der ersten flüssigen Phase des Gitters ein Interkalat bilden und auf diese Weise die optischen Eigenschaften des Gitters verändern.

In einer weiteren Ausgestaltung werden Moleküle eingesetzt, die durch Reaktion mit der flüssigen Phase aus dem Gitter eine chemische Verbindung bilden, deren Vorhandensein die optischen Eigenschaften des Gitters ändert. Bevorzugt sind organische Moleküle, insbesondere Dämpfe aus Alkoholen oder anderen leichtflüchtigen Substanzen, Tenside, oder Biomoleküle, insbesondere Proteine und Nukleinsäuren.

Schließlich werden während Schritt c) die durch die nachzuweisenden Moleküle hervorgerufenen Änderungen der Eigenschaften des mindestens einen optischen Beugungsgitters nachgewiesen. Der Nachweis erfolgt über eine Änderung der Intensität bzw. eine Änderung des Beugungsmusters, die sich bei der Reflexion oder der Transmission von Licht, bevorzugt aus einem Laser oder einer anderen kohärenten Lichtquelle, am optischen Gitter ergeben.

Dem Nachweis der Moleküle liegt die Idee zugrunde, eine Anzahl N an Informationen, d. h. die Konzentrationen N verschiedener Moleküle zu ermitteln, indem mindestens N Parameter durch Messungen ermittelt und man die Abhängigkeit jedes dieser Parameter als Funktion der Konzentration jedes der nachzuweisenden Moleküle kennt. Die Kenntnis dieser Parameter als Funktion der Konzentration jeder der nachzuweisenden Moleküle wird dabei vorzugsweise durch Kalibriermessungen, wobei jeweils nur eine Molekülspezies im Analyten vorhanden ist und alle N Parameter als Funktion der Konzentration dieser Spezies gemessen werden, gewonnen.

Zum *unspezifischen Nachweis* wird davon ausgegangen, dass die nachzuweisenden Moleküle bekannt sind und nur ihre Konzentration oder ihr Vorhandensein, d.h. der Nachweis der Konzentration oberhalb einer Nachweisgrenze, festzustellen ist. Hierbei ist es möglich, dass mehrere molekulare Spezies gleichzeitig nachgewiesen werden, ohne diese unterscheiden zu können.

Vorzugsweise bildet die Konzentration der nachzuweisenden Moleküle in der zweiten flüssigen Phase ein Maß für die Änderung der optischen Eigenschaften des optischen Gitters. Auf diese Weise wird nicht nur ein Nachweis der gesuchten molekularen Spezies, sondern auch die Untersuchung ihrer Konzentration und der Zeitabhängigkeit der Konzentration über die Beobachtungsdauer möglich.

Zum *spezifischen Nachweis* sieht das erfindungsgemäße Verfahren bevorzugt die folgenden Ausgestaltungen vor.

In einer ersten Ausgestaltung wird das Konzept der *spezifischen Bin*dung, insbesondere nach dem biochemischen *Schlüssel-Schloss-Prinzip* bzw. dem *fit-induced fit-Prinzip* eingesetzt. Hierzu werden in das mindestens eine optische Gitter oder in die zweite flüssigen Phase weitere Moleküle eingebracht, die mit den nachzuweisenden Molekülen derart eine spezifische Bindung eingehen, dass sich hierbei die optischen Eigenschaften des optischen Gitters so verändern, dass die spezifische Bindung den Nachweis der nachzuweisenden Moleküle ermöglicht.

In einer alternativen Ausgestaltung wird für den Nachweis eine Vielzahl von optischen Gittern eingesetzt, wobei sich die einzelnen optischen Gitter in mindestens einer Eigenschaft unterscheiden (so genannter *Chemischer Fingerprint).* Hierfür eignen sich insbesondere die für das Gitter eingesetzte erste flüssige Phase, die ausgewählte zweite flüssige Phase, Additive in der für das Gitter eingesetzten ersten flüssigen Phase, Additive in der ausgewählten zweiten flüssigen Phase, bzw. das Material, die Beschichtung, das Beschichtungsmuster oder die Rauhigkeit des Substrats.

Insbesondere sind hierzu mindestens zwei optische Gitter vorhanden, worin sich die periodischen Strukturen in ihrer Höhe unterscheiden.

Dadurch lässt sich die Reaktion des optischen Gitters auf das nachzuweisende Molekül über die Höhe des Gitters kontrollieren.

In einer weiteren Ausgestaltung wird das Konzept des so genannten *Physikalischen Fingerprints* eingesetzt. Hierzu wird dasselbe Gitter unter verschiedenen Winkeln bzw. bei verschiedenen Wellenlängen ausgelesen. Alternativ wird eine weitere physikalische Eigenschaft, insbesondere der Brechungsindex, für verschiedene Parameterwerte einer physikalischen Variablen ausgelesen. Vorteilhaft hierbei ist die Notwendigkeit nur eines einzelnen optischen Gitters.

Insbesondere wird eine Vielzahl von optischen Gittern mit derselben Lichtquelle (Laser) beaufschlagt und mit demselben Detektorsystem ausgelesen. Hierfür eignen sich Vielkanaldetektorsysteme wie z.B. ein CCD-Chip (paralleles Auslesen) bzw. Laserscanning und/oder Multiplexing (sequentielles Auslesen).

Mit dem erfindungsgemäßen Verfahren wird ein schnelles, einfach zu handhabendes, preiswertes und ohne Spezialkenntnisse durchzuführendes Nachweisverfahren mit der Möglichkeit des spezifischen Nachweises und der biomolekularen Erkennung vorgeschlagen. Vorteilhaft ist, dass sich über eine einfache optische Auslesetechnik mit handelsüblichen miniaturisierten Scannern viele Kanäle parallel oder sequentiell lesen lassen, womit eine Minaturisierung bis hinab zur Mikrometerskala und damit der Längenskala biologischer Zellen möglich wird.

Wesentlich für die Spezifizität des Nachweises ist die Möglichkeit, Standardverfahren zur biomolekularen Erkennung einzusetzen und die hieraus hervorgerufenen Änderungen der Beugungseigenschaften des flüssigen Gitters auf einfache Weise, schnell und zeitaufgelöst und mit hoher Empfindlichkeit nachzuweisen.

Der optische Nachweis eröffnet weiterhin die Möglichkeit, über optisch transparente Wände, z.B. aus Glas oder PMMA, in Echtzeit in geschlossenen Gefäßen Untersuchungen der Konzentration sowie spezifische Nachweise molekularer Spezies zu erbringen. Damit lassen sich in einem geschlossenen Gefäß kontaktlos von außen mit berührungslosem Scannen viele parallel arbeitender Sensoren (*Remote Reading*) einsetzen.

Die Erfindung wird im Folgenden anhand mehrerer Ausführungsbeispiele und den Figuren näher erläutert.

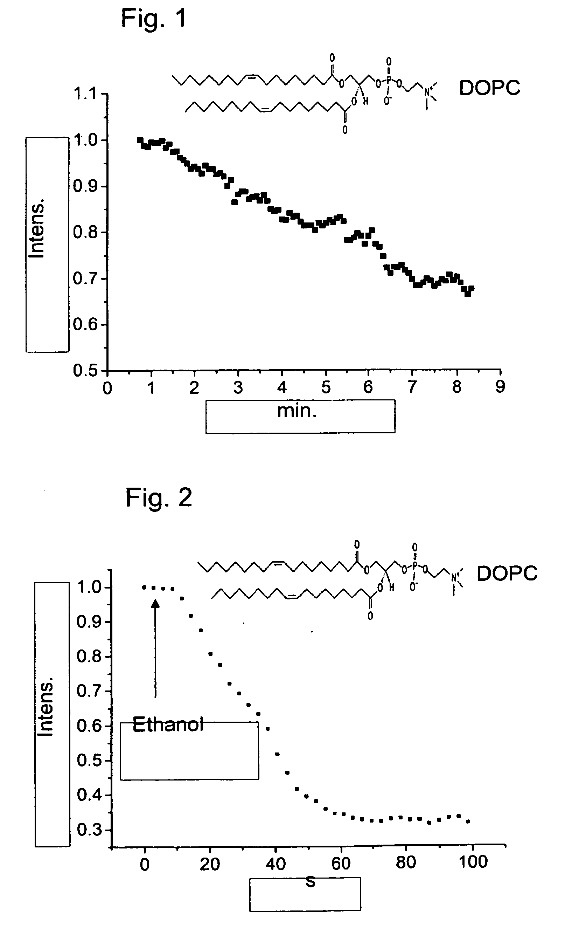

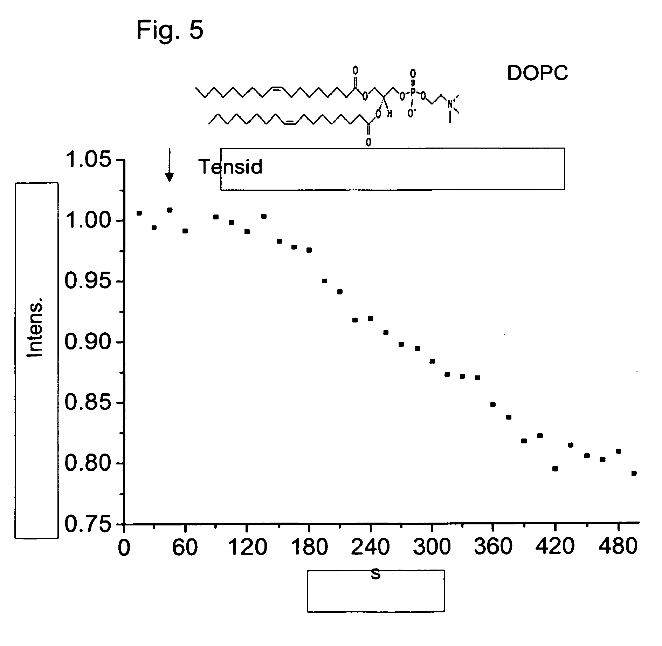

Für die Ausführungsbeispiele gemäß **Fig. 1-5** wurden optische Gitter aus lyotropischen Flüssigkristallen mit einer Periode von 500-800 nm eingesetzt, bestehend aus regelmäßigen Strukturen des Phospholipids 1,2-Dioleoyl-sn-Glycero-3-Phosphocholin (DOPC), die mittels *Dip-Pen Nanolithography* (DPN) auf Oberflächen aus Polymethylmethacrylat (PMMA), die zuvor jeweils für 5 Minuten unter Isopropanol und ultrareinem Wasser mit Ultraschall behandelt worden waren, aufgebracht wurden.

Zum Nachweis des Vorhandenseins der in Kontakt mit dem Gitter gebrachten Moleküle wurde das Gitter mit weißem Licht unter einem Winkel von ca. 70° zur Oberfläche beaufschlagt. Das normal zur Oberfläche gebeugte Licht wurde mittels einer Farb-CCD-Kamera aufgenommen.

**Figur 1** zeigt eine *Vergleichsmessung,* worin in willkürlichen Einheiten die Intensität des von einem derartigen optischen Gitter, das sich in Luft befindet, gebeugten Lichts gegenüber der Zeit aufgetragen ist, ohne dass hierauf nachzuweisende Moleküle aufgetragen wurden. Aufgrund der Luftfeuchtigkeit nimmt die beobachtete Intensität mit der Zeit monoton ab.

In **Fig. 2-5** ist jeweils die Intensität des gebeugten Lichts in willkürlichen Einheiten gegenüber der Zeit aufgetragen. Die Pfeile bezeichnen jeweils den Zeitpunkt, zu dem das Gitter Dämpfen aus bzw. Lösungen mit den jeweils bezeichneten Molekülen ausgesetzt wurde:

In **Fig. 2** wurden zum Zeitpunkt 0 s Ethanol-Dämpfe zugegeben.

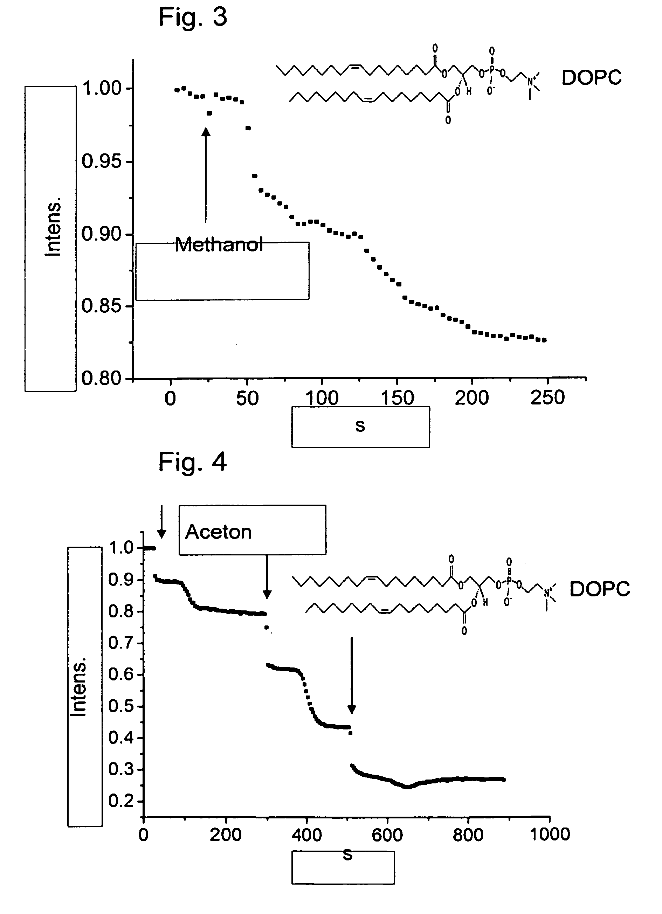

**In** **Fig. 3** wurden zum Zeitpunkt 25 s Methanol-Dämpfe zugegeben.

In **Fig. 4** wurden zu den Zeitpunkten 0 s, 250 s und 500 s jeweils Aceton-Dämpfe zugegeben.

In **Fig. 5** wurde eine 5 nM Lösung des Tensids Triton-X 100 zum Zeitpunkt 50 s zugegeben, wobei das Gitter **vor** der Zugabe des Tensids für mind. 5 Minuten unter Stickstoff-Atmosphäre in reines Wasser getaucht wurde. Während dieser Zeit veränderte sich die Intensität des gebeugten Lichts nicht wesentlich.

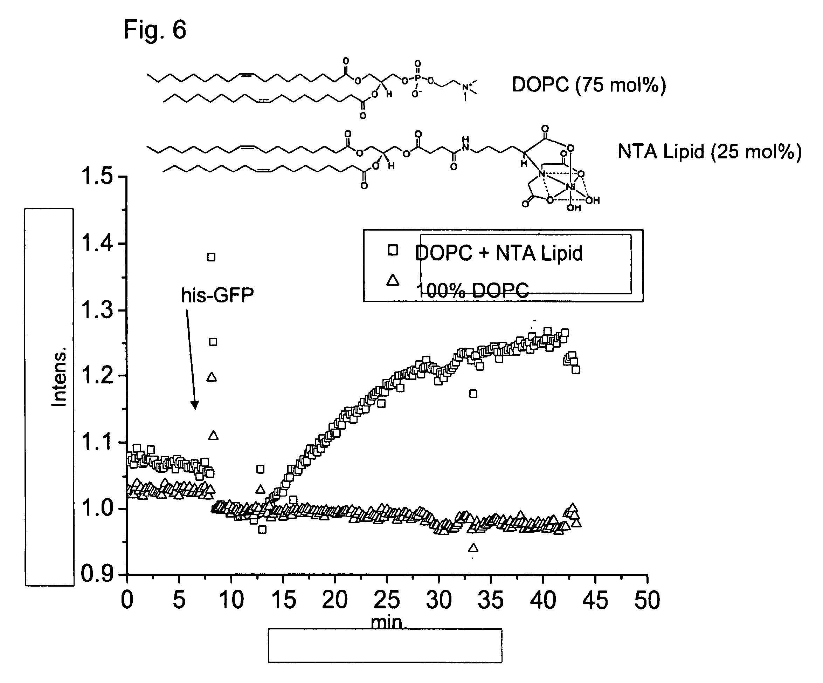

Zu dem in **Fig. 6** dargestellten Versuch wurden zu dem mit einem Pfeil gekennzeichneten Zeitpunkt eine 50 nM Lösung des Proteins *Polyhistidine tagged green fluorescent protein,* kurz his-GFP, mit dem Gitter in Kontakt gebracht. Hierbei wurde jedoch nur der rote Anteil des gebeugten Lichts zum Nachweis eingesetzt, da das Protein his-GFP im grünen Spektralbereich emittiert und eine Interferenz mit dem Grünanteil der Intensität des gebeugten Licht ausgeschlossen werden sollte.

Das optische Gitter wurde wie in **Fig. 1-4** hergestellt, jedoch wurde in **Fig. 6** eine Mischung aus 75 % DOPC und 25 % NTA Lipid, dessen Struktur in **Fig. 6** dargestellt ist, als Material für das optische Gitter eingesetzt. Darüber hinaus wurde zum Vergleich ein optisches Gitter aus reinem DOPC auf derselben Oberfläche hergestellt, um die Spezifität des Nachweises zu demonstrieren. Die optischen Gitter wurden nach ihrer Fertigstellung in eine Lösung aus Phosphat gepuffertem Silan, die 0,5 Gew.% BSA (*bovine serum albumin*) enthielt, getaucht, um eine nicht-spezifische Bindung zu verhindern. In **Fig. 6** sind jeweils die Intensitäten des gebeugten Lichts in willkürlichen Einheiten aus dem aus der Mischung bestehenden optischen Gitter durch **Quadrate** und dem aus reinem DOPC bestehenden optischen Gitter durch **Dreiecke** dargestellt.

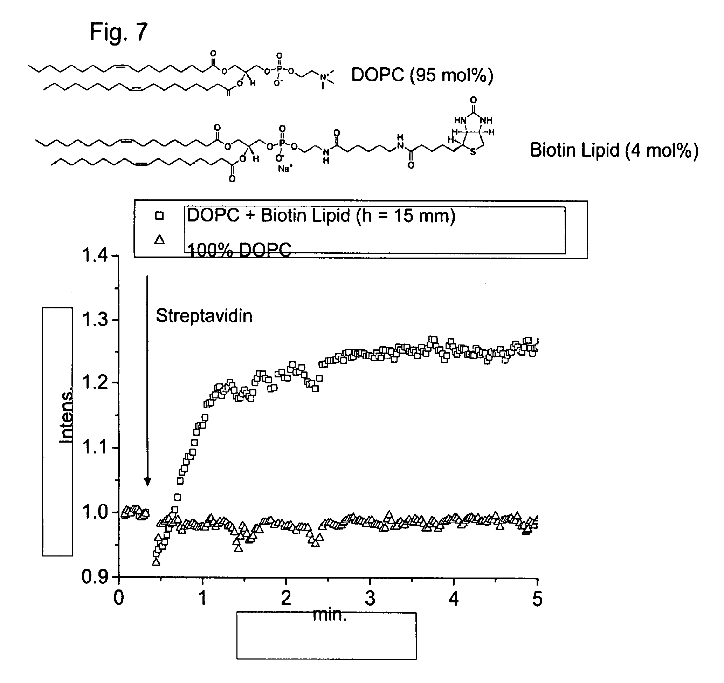

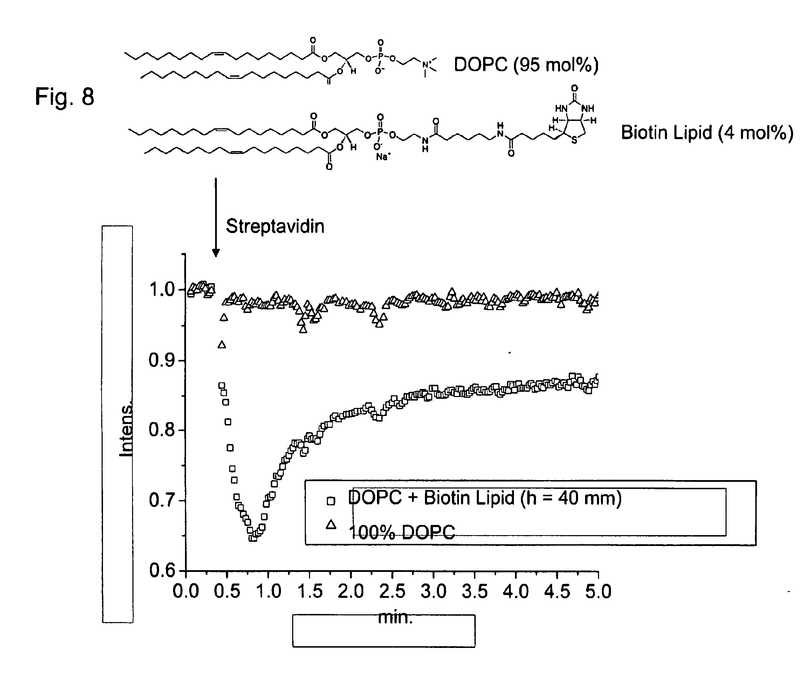

**In** **Fig. 7** **und** **8** wurde jeweils zu dem mit einem Pfeil gekennzeichneten Zeitpunkt eine 50 nM Lösung des Proteins Streptavidin mit dem Gitter in Kontakt gebracht. Das optische Gitter wurde wie in **Fig. 1-4** hergestellt, jedoch wurde in **Fig. 7** **und** **8** eine Mischung aus 95 % DOPC und 5 % Biotinyliertes Lipid, kurz Biotin Lipid, dessen Struktur in den **Fig. 7** **und** **8** dargestellt ist, als Material für das optische Gitter eingesetzt. Auch hier wurde zum Vergleich ein optisches Gitter aus reinem DOPC auf derselben Oberfläche hergestellt, um die Spezifität des Nachweises zu demonstrieren. Die betreffenden optischen Gitter wurden auch hier nach ihrer Fertigstellung in eine Lösung aus Phosphat gepuffertem Silan, die 0,5 Gew.% BSA enthielt, getaucht, um eine nicht-spezifische Bindung zu verhindern.

In **Fig. 7** **und** **8** sind jeweils die Intensitäten des gebeugten Lichts in willkürlichen Einheiten aus dem aus der Mischung bestehenden optischen Gitter durch **Quadrate** und dem aus reinem DOPC bestehenden optischen Gitter durch **Dreiecke** dargestellt.

Der Unterschied zwischen **Fig. 7** **und** **8** bestand darin, dass in **Fig. 7** die Höhe des optischen Gitters ca. 15 nm und in **Fig. 8** ca. 40 nm betrug. Der deutlich unterschiedliche Verlauf der Beugungsintensitäten über der Zeit demonstriert die Möglichkeit, die Höhe der Gitterstrukturen zur Einstellung der Reaktion des optischen Gitters auf das nachzuweisende Molekül einzusetzen. In **Fig. 7** reagierte das optische Gitter vorwiegend auf die Interkalation des Analyten, während in **Fig. 8** die Intensitätsabnahme auf die Entnetzung der Gitterelemente vom Substrat aufgrund der Anwesenheit des Analyten zurückzuführen war.

## Patentansprüche

1. Verfahren zum Nachweis des Vorhandenseins von Molekülen, mit den Schritten
a) Inkontaktbringen mindestens eines optischen Gitters, das aus einer ersten flüssigen Phase besteht, wobei die erste flüssigen Phase in Form von periodischen Strukturen, deren Periodizität von 190 nm bis 10 µm beträgt, auf einem Substrat angeordnet ist, mit einer zweiten flüssigen Phase, die nicht mischbar mit der ersten flüssigen Phase ist,
b) Zugeben der nachzuweisenden Moleküle, die die Fähigkeit besitzen, die optischen Eigenschaften des mindestens einen optischen Gitters zu verändern, vor, während oder nach Schritt a) in die erste flüssige Phase oder in die zweite flüssige Phase,
c) Nachweisen der veränderten Eigenschaften des mindestens einen optischen Gitters, sobald die erste flüssigen Phase sowohl mit der zweiten flüssigen Phase als auch mit den nachzuweisenden Molekülen in Kontakt getreten sind, über eine Messung der veränderten Reflexion oder Transmission von Licht an dem mindestens einen optischen Gitter oder des sich verändernden räumlichen Verlaufs des Beugungsmusters.

2. Verfahren nach Anspruch 1, wobei das mindestens ei:ne optische Gitter mittels Dip-Pen Nanolithographie, Mikropipetten, Mikrokontaktdruck, durch strukturierte Entnetzung der ersten flüssigen Phase auf dem Substrat oder durch Schmelzen eines lithographisch strukturierten Feststoffs hergestellt wird.

3. Verfahren nach Anspruch 1 oder 2, wobei das mindestens eine optische Gitter auf ein Substrat aufgebracht wird, das Oberflächenrauheiten, Topographien, chemische Heterogenitäten oder geometrische Strukturierungen aufweist, woran sich die Strukturen aus der ersten flüssigen Phase festhalten.

4. Verfahren nach einem der Ansprüche 1 bis 3, wobei die Strukturen des optischen Gitters aus einem Membranlipid bestehen und als zweite flüssige Phase eine wässrige Lösung, worin die nachzuweisenden Moleküle enthalten sind, eingesetzt wird.

5. Verfahren nach einem der Ansprüche 1 bis 4, wobei in die erste flüssige Phase, aus der das optische Gitter besteht, weitere Moleküle eingebracht werden, die mit den nachzuweisenden Molekülen derart eine spezifische Bindung eingehen, dass sich hierbei die optischen Eigenschaften des optischen Gitters verändern.

6. Verfahren nach einem der Ansprüche 1 bis 5, wobei mindestens ein optisches Gitter unter verschiedenen Winkeln oder bei verschiedenen Wellenlängen ausgelesen wird.

7. Verfahren nach einem der Ansprüche 1 bis 6, wobei mindestens zwei optische Gitter, die sich in ihren optischen Eigenschaften unterscheiden, eingesetzt werden.

8. Verfahren nach Anspruch 7, wobei mindestens zwei optische Gitter, deren periodischen Strukturen sich in ihrer Höhe unterscheiden, eingesetzt werden.

9. Verfahren nach einem der Ansprüche 1 bis 8, wobei organische Moleküle oder Biomoleküle als nachzuweisende Moleküle eingesetzt werden.

10. Verfahren nach einem der Ansprüche 9, wobei Tenside, Proteine oder Nukleinsäuren als nachzuweisende Moleküle eingesetzt werden.

## Claims

1. Method for demonstrating the presence of molecules, comprising the steps
a) bringing at least one optical grating which consists of a first liquid phase, wherein the first liquid phase is arranged on a substrate in a form of periodic structures having a periodicity of from 190 nm to 10 µm, into contact with a second liquid phase which is immiscible with the first liquid phase,
b) adding the molecules to be demonstrated, which have the ability to modify the optical properties of the at least one optical grating, to the first liquid phase or to the second liquid phase before, during or after step a),
c) detecting the modified properties of the at least one optical grating as soon as the first liquid phase is brought into contact both with the second liquid phase and with the molecules to be demonstrated, by means of a measurement of the modified reflection or transmission of light at the at least one optical grating or the changing spatial progression of the diffraction pattern.

2. Method according to claim 1, wherein the at least one optical grating is produced by means of dip-pen nanolithography, micro-pipettes, micro-contact pressure, by structured dewetting of the first liquid phase on the substrate or by melting a lithographically structured solid.

3. Method according to claim 1 or 2, wherein the at least one optical grating is applied to a substrate which has surface roughnesses, topographies, chemical heterogeneities or geometric structurings to which the structures of the first liquid phase adhere.

4. Method according to any one of claims 1 to 3, wherein the structures of the optical grating consist of a membrane lipid, and an aqueous solution which contains the molecules to be demonstrated is used as the second liquid phase.

5. Method according to any one of claims 1 to 4, wherein incorporated in the first liquid phase, from which the optical grating is made, are further molecules which specifically bind to the molecules to be demonstrated such that the optical properties of the optical grating are thereby modified.

6. Method according to any one of claims 1 to 5, wherein at least one optical grating is read at different angles or at different wavelengths.

7. Method according to any one of claims 1 to 6, wherein use is made of at least two optical gratings which differ in terms of their optical properties.

8. Method according to claim 7, wherein use is made of at least two optical gratings, the periodic structures of which differ in terms of their height.

9. Method according to any one of claims 1 to 8, wherein organic molecules or biomolecules are used as the molecules to be demonstrated.

10. Method according to claim 9, wherein surfactants, proteins or nucleic acids are used as the molecules to be demonstrated.

## Revendications

1. Procédé pour détecter la présence de molécules comprenant les étapes consistant à :
a) mettre en contact au moins un réseau optique constitué par une première phase liquide, cette première phase liquide étant appliquée sur un substrat sous la forme de structures périodiques dont la périodicité est de 190 nm à 10 µm, avec une seconde phase liquide qui n'est pas miscible avec la première phase liquide,
b) ajouter les molécules à détecter qui présentent la faculté de modifier les propriétés optiques du réseau optique pendant ou après l'étape a), dans la première phase liquide ou dans la seconde phase liquide,
c) détecter les propriétés modifiées du réseau optique dès que la première phase liquide a été mise en contact avec la seconde phase liquide et également avec les molécules à détecter, par mesure de la modification de la réflexion ou de la transmission de la lumière sur le réseau optique ou de la variation spaciale du modèle diffraction.

2. Procédé conforme à la revendication 1, selon lequel le réseau optique est obtenu par nano-lithographie dip-pen, micropipettes, impression par microcontact, par déréticulation structurée de la première phase liquide sur le substrat, ou par fusion d'un corps solide structuré par lithographie.

3. Procédé conforme à la revendication 1 ou 2, selon lequel le réseau est appliqué sur un substrat présentant des rugosités de surface, des topographies, des hétérogénéités chimiques ou des structurations géométriques, sur lesquelles sont maintenues les structures de la première phase liquide.

4. Procédé conforme à l'une des revendications 1 à 3, selon lequel les structures du réseau optique sont constituées par un lipide membranaire, et, en tant que seconde phase liquide on utilise une solution aqueuse renfermant les molécules à détecter.

5. Procédé conforme à l'une des revendications 1 à 4, selon lequel on introduit dans la première phase liquide constituant le réseau optique d'autres molécules qui forment avec les molécules à détecter une liaison spécifique entrainant une modification des propriétés optiques du réseau optique.

6. Procédé conforme à l'une des revendications 1 à 5, selon lequel au moins un réseau optique est exploité sous différents angles ou à différentes longueurs d'ondes.

7. Procédé conforme à l'une des revendications 1 à 6, selon lequel on utilise au moins deux réseaux optiques dont les propriétés optiques sont différentes.

8. Procédé conforme à la revendication 7, selon lequel on utilise au moins deux réseaux optiques dont les hauteurs des structures périodiques sont différentes.

9. Procédé conforme à l'une des revendications 1 à 8, selon lequel on utilise des molécules organiques ou des biomolécules entant que molécules à détecter.

10. Procédé conforme à la revendication 9, selon lequel on utilise des agents tensio-actifs, des protéines ou des acides nucléiques en tant que molécules à détecter.
